# EUROPEAN PATENT APPLICATION

(11) **EP 4 104 742 A1**
(43) Date of publication of application: **21.12.2022**
(21) Application number: 21179543.0
(22) Date of filing: 15.06.2021
(51) Int. Cl.: A61B 1/00, A61B 1/07, A61B 1/06, G02B 23/24

(54) **AN ENDOSCOPE**

(71) Applicant: Ambu A/S, 2750 Ballerup (DK)
(72) Inventor: JOHNSEN, Lasse Markworth, 3460 Birkerød (DK); SØRENSEN, Morten, 2750 Ballerup (DK); LUNDBECH, Michael René, 4100 Ringsted (DK)
(74) Representative: AWA Denmark A/S

(57) **Abstract**

An endoscope with a distal tip part comprising a tip housing integrally moulded from a first material and a second material. The housing comprises an end wall (10) and a surrounding side wall (11) so as to define an inner compartment accommodating an electronic vision device and at least one light source. The end wall (10) comprises an end surface and the surrounding side wall (11) comprises a surrounding side surface.

The second material is provided as one contiguous part providing part of the side wall (11) and part of the end wall (11) so as to provide a vision window (13) in the end wall covering the electronic device and a light emission window (14) adapted to receive and transmit light from the at least one light source through the end wall (10). In the end wall (10), said second material overlaps at least partially said first material when viewed from the end surface.

## Description

The present invention relates to insertable medical vision devices, in particular disposable insertion endoscopes, and more specifically to a housing for the tip of the disposable insertion endoscope and the manufacture thereof.

Vision devices, such as insertion endoscopes are well known devices for visually inspecting body cavities, such as human body cavities. Typically, an insertion endoscope comprises an elongated insertion tube with a handle at the proximal end as seen from the operator and visual inspections means, such as a built-in camera, at the distal end of the elongated insertion tube. Electrical wiring for the camera and other electronics such as LED lighting accommodated in the tip part at the distal end run along the inside of the elongated insertion tube from the handle to the tip part. Instead of using cameras, endoscopes may also be fibre-optic, in which case the optical fibres run along inside of the elongated insertion tube to the tip part.

In order to be able to manoeuvre the endoscope inside the body cavity, the distal end of the endoscope may comprise a bending section with increased flexibility, e.g. a number of articulated segments of which the tip part forms the distalmost segment. This is typically done by tensioning or slacking pull wires also running along the inside of the elongated insertion tube from the tip part through the remainder of articulated segments to a control mechanism of the handle. Furthermore, a working channel may run along the inside of the insertion tube from the handle to the tip part, e.g. allowing liquid to be removed from the body cavity or allowing the insertion of surgical instruments or the like into the body cavity.

As the name indicates, endoscopes, are used for seeing inside things, such as lungs or other human body cavities of a patient. Modern endoscopes are therefore typically equipped with a least one camera or similar image capturing device at the distal tip of the endoscope. Provided that sufficient light is present, this allows the operator to see where the endoscope is steered and to set the target of interest once the tip has been advanced thereto. This therefore normally requires illumination of the area in front of the distal tip of the endoscope, in particular the field of vision of the camera(s). One known way of achieving such illumination is to provide the above mentioned LED lighting using one or more Light Emitting Diodes (LEDs) in the tip of the endoscope, as e.g. mentioned in WO2014/106511 disclosing a disposable endoscope.

When, as according to the present disclosure, the insertion tube of the endoscope is intended to be inserted into a human body cavity, the insertion tube needs to be sealed in a watertight manner. This is in particular the case for the distal tip part because it accommodates the camera, LED(s) and other delicate electronics, prone to malfunction or destruction if exposed to humidity. Also, there are electrical requirements to ensure that the electrical insulation of the endoscope is not compromised e.g. by electrical breakdown of the polymer material from which disposable endoscopes are typically made.

Furthermore, it is desirable to provide the front window of the tip part with shading members through two-stage two-component injection moulding of a transparent window material and an opaque shading material. This is to minimize glare from the built-in light sources to the camera of the insertion endoscope. One such tip part and manufacturing method is known form EP3539449, incorporated herein by reference.

While the tip housing in accordance with EP3539449 has shown to generally fulfil the above requirements the joining of the two materials is not always optimal, so that the electrical insulation becomes sub-standard, in turn leading to rejects during subsequent testing of the endoscopes, in turn, causing unnecessary costs.

Based on this prior art it is an object of the invention to provide an endoscope with an improved housing for the tip part which does not suffer from the above drawbacks.

According to a first aspect of the disclosure this object is achieved by an endoscope with a distal tip part comprising a tip housing integrally moulded from a first material and a second material, the second material being a transparent material, said housing comprising an end wall and a surrounding side wall so as to define at least one inner compartment accommodating an electronic vision device and at least one light source, where the end wall comprises an end surface and the surrounding side wall comprises a surrounding side surface, where the second material is provided as a part of the end wall so as to provide a vision window in the end wall covering the electronic vision device and a light emission window adapted to receive and transmit light from the at least one light source through the end wall, wherein, in the end wall, said second material overlaps at least partially said first material when viewed from the end surface.

By creating such an overlap, the area over which the first and second materials contact and fuse together is increased, thus better ensuring proper fusion of the two materials. Furthermore, the distance along the fusion seam from the inside to the outside is increased, in turn, leading to a longer electrical paths, should defects in the fusion exist, hence further decreasing the risk of electrical breakdown.

According to a second aspect the object is achieved by a system comprising a display device and an endoscope according to the first aspect of the disclosure connectable to the display device. In such a system the use of an endoscope according to the first aspect reduces the risk of electrical breakdown induced via the electrical connection between the endoscope and display device.

According to an embodiment according to the first aspect of the invention, the second material is provided as one contiguous part providing part of the side wall and part of the end wall. This allow the moulding inlet of the mould to be placed at a location away from front window so as to minimize any disturbance of the optical properties that may be caused by the moulding process.

According to an embodiment according to the first aspect of the disclosure, the overlap is formed as a step between the first and second materials. Using a step further increase the distance from the inside to the outside along the fusion seam and may provide large interface areas where the first and second material fuse together.

According to another embodiment according to the first aspect of the disclosure, the step comprises an interface extending below and in parallel with the end surface. Having the interface extending below the end surface, increases the mechanical resistance of the fusion seam as the main forces acting on the end surface during insertion of the endoscope will be perpendicular to the interface.

According to a further embodiment according to the first aspect of the disclosure, the second material overlaps a moulding artefact in the first material. Thereby, moulding artefacts, such as defects and remnants from the inlet of the first molding stage may be covered, in turn, providing the end wall with a smooth surface on which nothing may get caught and which is better at repelling residue that may block the vision or illumination.

According to yet another embodiment according to the first aspect of the disclosure, the tip is injection moulded in a two-stage injection moulding process in which, in the first stage, the first material is injected into a mould through a first single inlet and in which, in the second stage said second material overlap is injected through a second single inlet.

According to yet a further embodiment according to the first aspect of the disclosure, the second inlet is arranged in conjunction with the side surface of the tip housing. This allows proper flow of the second material from the second inlet into the front window part and the light guides which are preferably arranged mirror-symmetrical on either side of the central vision window, ensuring good filling and hence good optical properties, inter alia by keeping the single inlet away from the front window surfaces.

According to still another preferred embodiment, the first inlet is arranged in conjunction with the interface. This allows central filling and good flow from a single inlet that may later be covered by the second material injected elsewhere.

The disclosure will now be made in greater detail based on non-limiting exemplary embodiments and with reference to the drawings on which:
Fig. 1 shows a system comprising a display device and an endoscope according to the disclosure connected thereto,
Fig. 2 shows a tip housing used in the endoscope according to the disclosure,
Fig. 3 shows an exploded view of the tip housing of Fig. 2,
Fig. 4 shows a distal end view of the tip housing of Fig. 2,
Fig. 5 shows the inside of the tip housing of Fig. 2 as seen from the proximal end,
Fig. 6 shows a perspective front view of the first housing part,
Fig. 7 schematically shows the first housing part in a longitudinal section of a first configuration of an exemplary mould, and
Fig. 8 schematically shows the complete housing part in a longitudinal section of a second configuration of the exemplary mould.

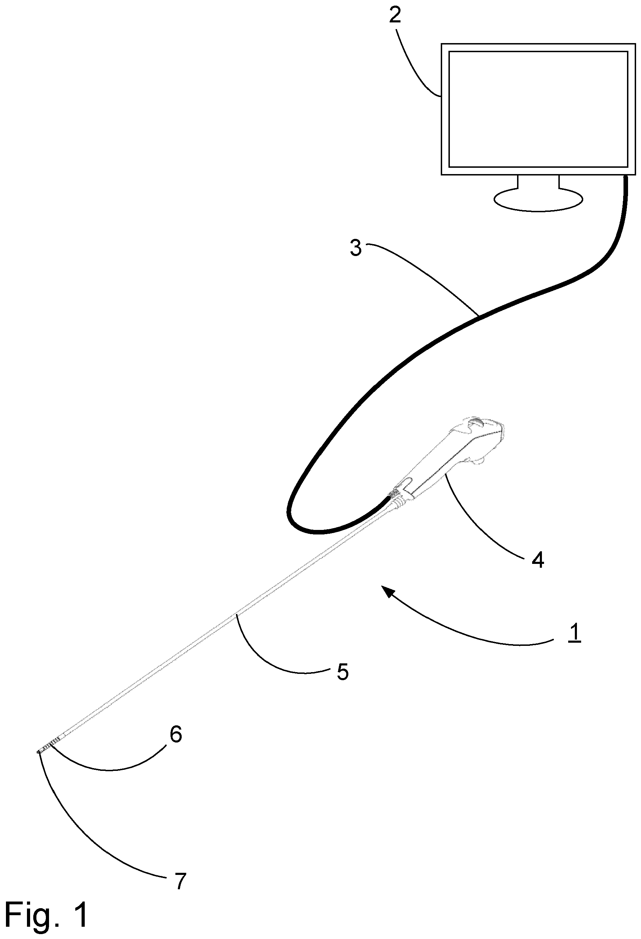

Turning first to Fig. 1 a system comprising an endoscope 1 according to the disclosure and a display device 2 is shown. The endoscope 1 is connected to the display device 2 via a communications connection, which in the illustrated example is a cable 3. The cable 3 may also include power supply for the electronics in the endoscope 1 as well as channels for irrigation and suction etc. thus constituting what is commonly referred to as an umbilical.

The endoscope 1 in the illustrated example comprises a proximal handle 4 adapted to be gripped by a hand of an operator. From the handle 4 an insertion cord 5 extends towards the distal end of the endoscope 1. At the distal end of the endoscope 1 the insertion cord 4 is connected to a highly flexible bending section 6 which includes a tip housing 7, typically constituting the most distal end of the endoscope 1. The focus of the present disclosure is on the tip housing 7, as illustrated in the subsequent figures, and the skilled person will understand that the remainder of the endoscope 1 may have a different design without deviating from the essence of the disclosure.

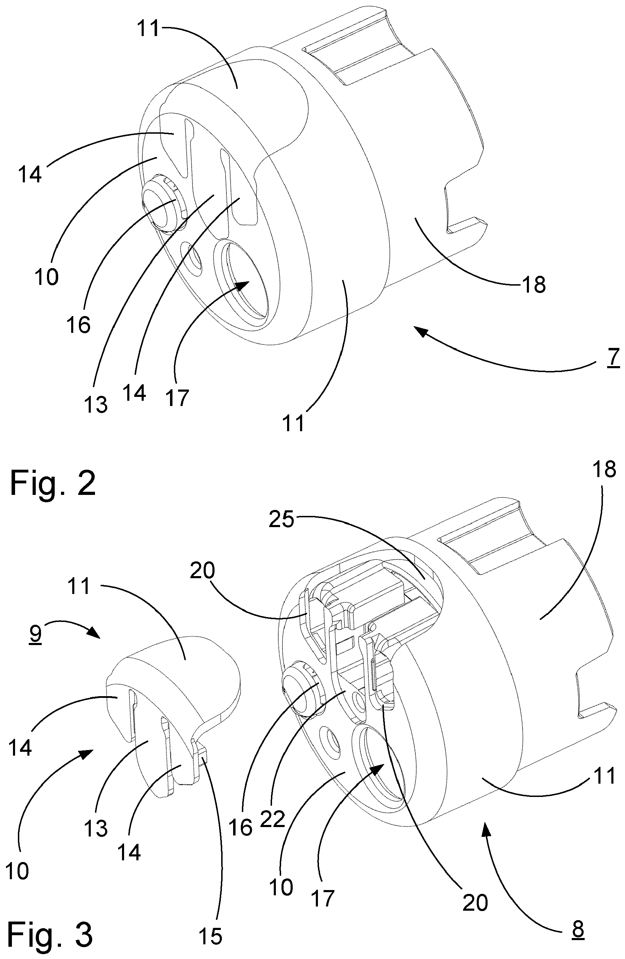

In Fig. 2 the tip housing 7 is shown in greater detail. The tip housing 7 is integrally moulded from a first material and a second material, so as to comprise a first housing part 8 and a second housing part 9. The second material forming the second housing part 9 is a transparent material. The first material forming the first housing part on the other hand is coloured or preferably opaque. The first and second materials are preferably polymer materials and suitable for injection moulding. The polymer of the first and second materials may be the same, i.e. the materials differing only in the filler, such as carbon black, coloured dye or lack of same.

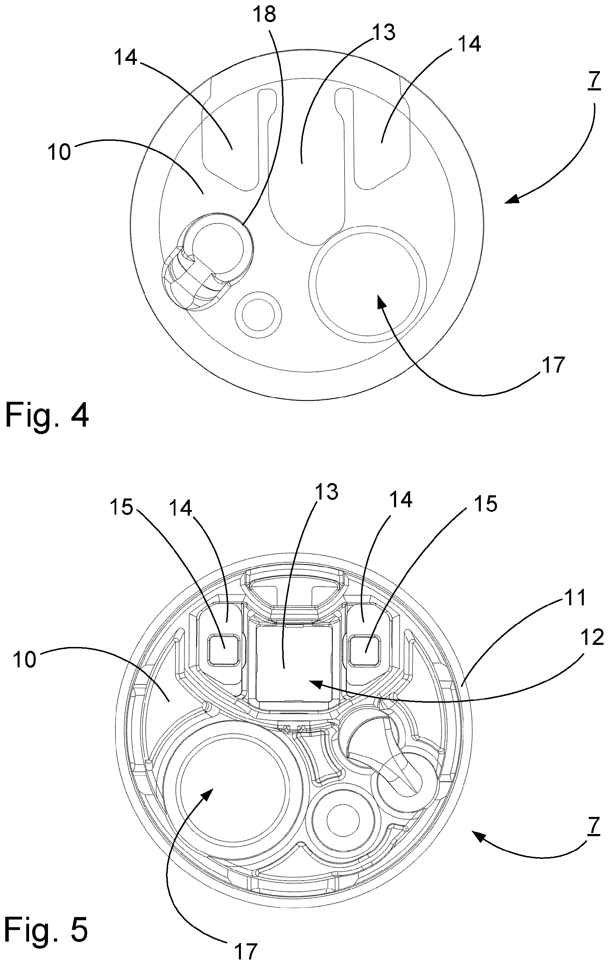

The housing 7 comprises an end wall 10 and a surrounding side wall 11 so as to define at least one inner compartment 12, best seen in Figs. 3, 5 and 6. The inner compartment accommodates an electronic vision device and at least one light source, not shown. The end wall 10 comprises an end surface and the surrounding side wall 11 comprises a surrounding side surface, forming the exterior surface of the housing 7.

As can best be seen from Fig. 3, the second material is provided as one contiguous part providing part of the side wall 11 and part of the end wall 10 so as to provide a vision window 13 in the end wall covering the electronic device (not shown) and at least one light emission window 14. In the illustrated example there are two light emission windows 14 arranged mirror-symmetrically on either side of the vision window 13. The light emission windows 14, may comprise a light guide 15 adapted to receive and transmit light from the at least one light source (not shown) through the end wall 10. The light guides 15 are adapted to shape the light emitted from the light sources in order to provide a desired light distribution withing the field of view of the endoscope 1, e.g. as described in EP3539451, incorporated herein by reference.

The end wall 10 may comprise additional features provided in the first material such as a spray nozzle 16 and a working channel exit port 17 where the spray nozzle is adapted to spray water onto the vision window 13 and/or the light emission windows 14.

Towards the proximal end of the housing 7 the surrounding side surface may have a slightly recessed part 18 to allow the bending section 6 to overlap the housing 7 and form a good connection when assembling the endoscope 1. On top of both the housing 7 and the bending section 6 an outer sleeve or covering is normally fitted, in order to avoid undesired fluid ingress into the bending section 6, which is normally a relatively open articulated structure.

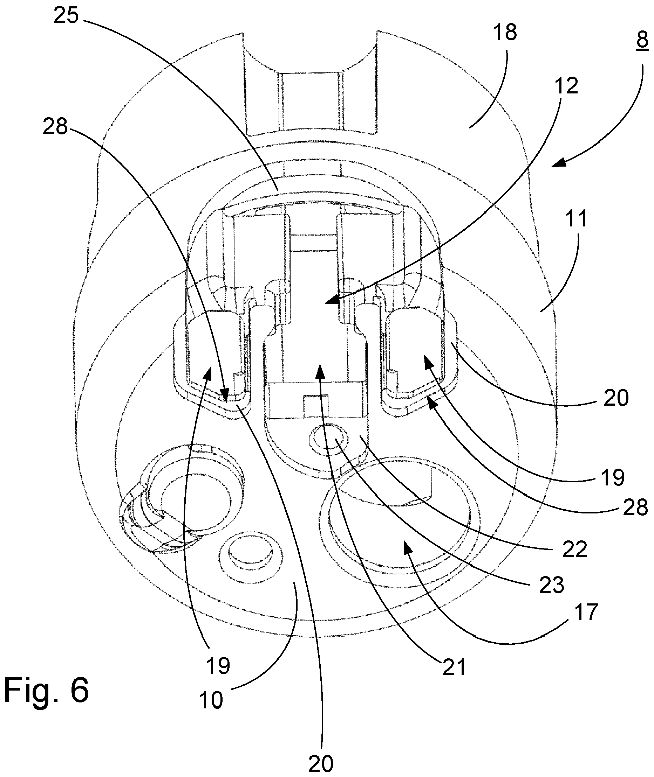

Turning now to Fig. 6 details of the first housing part 8 as it would appear after a first moulding stage, i.e. before the second material is added in a second moulding stage, can better be seen. Since the first material is preferably opaque, apertures 19 allowing the light from the light sources within the housing 7 are provided in the end wall 10. These apertures 19 will be covered by the light emission windows 14 when in the second moulding stage. The apertures 19 have a cross-sectional area and a circumference allowing for the accommodation of the light guides 15, if provided, e.g. as an integral part of the light emission window 14. As can be seen the end wall comprises a transition section 28 through the end wall so that the light emission window area at the end surface is larger than the cross-sectional area of the aperture deeper within the end wall 10. This could be provided by flaring the walls of the aperture 19 out, but preferably this is done in a stepwise manner as illustrated. This provides plane surfaces 20 within the end wall 10 of the final housing 7 after the second moulding step, so as to provide an increased adhesion or fusion area between the first material and the second material. The plane surfaces 20 are preferably arranged so that they are parallel with the final end surface 10 of the finished housing 7, thus also providing good manual support for the light emission windows 14. In other words the windows formed from the second material are provided with an undercut supported by the first material. Furthermore, the distance from the end surface outside the end wall 10 to the interior of the housing along the fusion seam through the wall, becomes greater than if the fusion seam was straight through the end surface perpendicular to the surface. In other words, the interface between the first material and the second material becomes larger because of the overlap. Should defects exist the path for ingress of fluids and electrical breakdown along the fusion seam will be longer, and the risk of fluid penetration through the end wall and the risk of electrical breakdown accordingly reduced.

Similarly, i.e. for the same reasons, the passage 21 for light into the vision device may also be widened towards the end surface 10, preferably also in a stepwise manner providing an essentially plane surface 22 adjacent the through passage 21. The plane surface 22 is preferably also arranged to be parallel to the final end surface 10 of the finished housing 7.

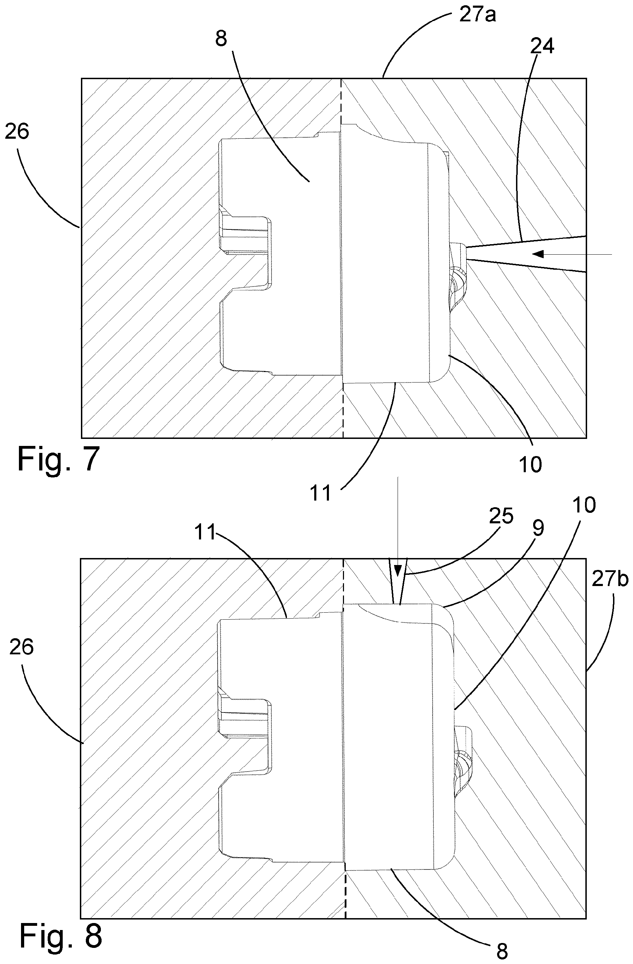

As can be seen the essentially plane surface 22 may comprise a defect in the form of remnants 23 of the inlet 24 (cf. Fig. 7) for the polymer material into the mould cavity in the first moulding step. This allows the first housing part 8 to be moulded using only a single inlet 24, the remnants of which may then be covered to provide a smooth end surface.

As will be further explained below and seen from Fig. 8 the transparent second housing part 9 may also be moulded using a single inlet 25 only. This inlet may be provided in the side wall 11 where defects from the inlet will not have any influence on the optical transmission properties of the light emission windows 14 and the vision window 13. Also, here a step may be provided in the wall 11 so as to provide a concentric surface 25 to support the second material and to increase the length of the fusion seam.

Turning now to Fig. 7, a longitudinal section through a first mould configuration for the first moulding stage is shown with the moulded first housing part 8 (not in section) placed in the mould cavity for illustration. Similarly, in Fig. 8 a longitudinal section of a second mould configuration for the second moulding stage is shown with the finished moulded housing 7 (not in section) placed in the mould cavity for illustration.

In Fig. 7 a first mould configuration comprising two separable halves 26, 27a forming a fist mould cavity corresponding to the first housing part 8, is shown. The first mould half 26 has a central convex shape that protruded into the cavity of the second half 27a so as to provide the interior volume of the first housing part 8 when moulded in the first stage of a two-stage two component injection moulding process. The first material is preferably injected through a single inlet arranged close to a centre axis of the first housing part 8. When the first material has set the two halves are separated, preferably with the already set first material preferably remaining stuck in the first half 26. A new second mould configuration shown in Fig. 8 may then be provided by substituting the second mould part 27a with new second half 27b corresponding to the desired final shape of the housing 7. Remaining stuck in the first half 26, the already set first part thus fills the reconfigured moulding cavity partially, leaving only room for the second material to provide the transparent second housing part 9 with the transparent windows 13, 14 of the final housing 7. The second material may then be injected via a single inlet so as to form a single contiguous part. This may be done with increased pressure so that the first material properly contacts the inner wall of the second mould part 27a and provides windows 13, 14 with no undesired defects. When the second material set the finished housing 7 may be removed from the mould.

Because the two single inlets 24 and 25 are in different locations the second material may smoothly cover any remnants of the first inlet of the mould part 27a in the finished housing 7.

## Claims

1. An endoscope with a distal tip part comprising a tip housing integrally moulded from a first material and a second material, the second material being a transparent material,
said housing comprising an end wall and a surrounding side wall so as to define at least one inner compartment accommodating an electronic vision device and at least one light source,
where the end wall comprises an end surface and the surrounding side wall comprises a surrounding side surface,
where the second material is provided as a part of the end wall so as to provide a vision window in the end wall covering the electronic vision device and a light emission window adapted to receive and transmit light from the at least one light source through the end wall,
wherein, in the end wall, said second material overlaps at least partially said first material when viewed from the end surface.

2. An endoscope according to claim 1, wherein the second material is provided as one contiguous part providing part of the side wall and part of the end wall.

3. An endoscope according to claim 1 or 2, wherein the overlap is formed as a step between the first and second materials.

4. An endoscope according to claim 1 or 2, wherein the step comprises an interface extending below and in parallel with the end surface.

5. An endoscope according to anyone of the preceding claims wherein said second material overlaps a moulding artefact in the first material.

6. An endoscope according to any one of the preceding claims wherein the tip is injection moulded in a two stage injection moulding process in which, in the first stage, the first material is injected into a mould through a first single inlet and in which, in the second stage said second material overlap is injected through a second single inlet.

7. An endoscope according to any one of claims 5 or 6, wherein said second material overlaps the location of said first inlet.

8. An endoscope according to claim 6 or 7, wherein the second inlet is arranged in conjunction with the side surface of the tip housing.

9. An endoscope according to any one of claims 5 to 8 when dependent on claim 4, wherein the first inlet is arranged in conjunction with the interface.

10. An endoscope according to any one of the preceding claims, wherein the light emission window comprises a light guide.

11. A system comprising a display device and an endoscope according to any one of claims 1 to 10 connectable to the display device.
